(19) **Europäisches Patentamt / European Patent Office / Office européen des brevets**

(11) **EP 4 471 401 A1**

(12) **EUROPEAN PATENT APPLICATION**
published in accordance with Art. 153(4) EPC

(43) Date of publication:
**04.12.2024 Bulletin 2024/49**

(21) Application number: **22923995.9**

(22) Date of filing: **29.09.2022**

(51) International Patent Classification (IPC):
**G01N 1/10** (2006.01)        **G01N 33/48** (2006.01)

(52) Cooperative Patent Classification (CPC):
**G01N 1/10; G01N 33/48**

(86) International application number:
**PCT/JP2022/036449**

(87) International publication number:
**WO 2023/145137 (03.08.2023 Gazette 2023/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.01.2022 JP 2022012202**

(71) Applicant: **Sekisui Medical Co., Ltd.**
**Tokyo 103-0027 (JP)**

(72) Inventors:
- **KOMAI, Kuniya**
  **Shunan-shi, Yamaguchi 746-0006 (JP)**
- **HOSSAIN, Md Shahadat**
  **Shunan-shi, Yamaguchi 746-0006 (JP)**
- **INOUE, Tomonori**
  **Shunan-shi, Yamaguchi 746-0006 (JP)**

(74) Representative: **Ter Meer Steinmeister & Partner Patentanwälte mbB**
**Nymphenburger Straße 4**
**80335 München (DE)**

(54) **BLOOD COLLECTION CONTAINER, METHOD FOR SEPARATING PLASMA, METHOD FOR SEPARATING EXTRACELLULAR FREE NUCLEIC ACID, AND METHOD FOR SEPARATING EXTRACELLULAR VESICLE**

(57) Provided is a blood collection container capable of suppressing contamination of plasma with leukocyte-derived DNA during specimen storage. A blood collection container according to the present invention includes a blood collection container main body, a plasma separation material contained in the blood collection container main body, and an aqueous solution contained in the blood collection container main body, wherein the plasma separation material has a specific gravity at 25°C of 1.027 or more and 1.060 or less, and the aqueous solution contains an anticoagulant, as well as contains a water-soluble polymer compound having a number average molecular weight of 500 or more and 180,000 or less or ammonium sulfate.

[FIG. 1]

EP 4 471 401 A1

**Description**

**TECHNICAL FIELD**

The present invention relates to a blood collection container. The present invention also relates to a method for separating plasma, a method for separating extracellular free nucleic acid, and a method for separating extracellular vesicle, the methods using the blood collection container.

**BACKGROUND ART**

In clinical examinations, blood collection containers such as blood collection tubes are widely used for collecting blood. After blood is collected in a blood collection container containing a plasma separation material, the blood collection container is centrifuged, whereby the blood can be separated into plasma and hemocytes. At this time, the plasma is located above the plasma separation material, and the hemocytes are located below the plasma separation material. As a blood collection container containing a plasma separation material, a blood collection container (for example, Patent Document 1) containing a plasma separation composition containing a resin and an inorganic powder, and a blood collection container (for example, Patent Document 2) containing a plasma separation jig are known.

**Related Art Documents**

**Patent Documents**

Patent Document 1: WO 2010/053180 A1
Patent Document 2: WO 2010/132783 A1

**SUMMARY OF THE INVENTION**

**PROBLEMS TO BE SOLVED BY THE INVENTION**

When plasma is separated from blood using a blood collection container containing a plasma separation material, leukocytes may be contaminated in the separated plasma.
Meanwhile, the separated plasma is stored for a certain period of time without being frozen until it is used for examination. In a conventional blood collection container as described in Patent Documents 1 and 2, leukocytes contaminated in plasma are destroyed over time due to death or the like, and DNA in the leukocytes may leak into the plasma. Leakage of DNA in leukocytes into plasma may affect the test result.
For example, in a test for detecting extracellular free nucleic acid (for example, cell free DNA) in plasma, the test result greatly varies depending on DNA leaking from leukocytes.
It is an object of the present invention to provide a blood collection container capable of suppressing contamination of plasma with leukocyte-derived DNA during specimen storage. It is also an object of the present invention to provide a method for separating plasma, a method for separating extracellular free nucleic acid, and a method for separating extracellular vesicle, the methods using the blood collection container.

**MEANS FOR SOLVING THE PROBLEM**

According to a broad aspect of the present invention, there is provided a blood collection container including a blood collection container main body, a plasma separation material contained in the blood collection container main body, and an aqueous solution contained in the blood collection container main body, wherein the plasma separation material has a specific gravity at 25°C of 1.027 or more and 1.060 or less, and the aqueous solution contains an anticoagulant, as well as contains a water-soluble polymer compound having a number average molecular weight of 500 or more and 180,000 or less or ammonium sulfate.
In a specific aspect of the blood collection container according to the present invention, the aqueous solution contains the water-soluble polymer compound having a number average molecular weight of 500 or more and 180,000 or less.
In a specific aspect of the blood collection container according to the present invention, the aqueous solution contains the ammonium sulfate.
In a specific aspect of the blood collection container according to the present invention, the aqueous solution contains the water-soluble polymer compound having a number average molecular weight of 500 or more and 180,000 or less, and the ammonium sulfate.

In a specific aspect of the blood collection container according to the present invention, the water-soluble polymer compound having a number average molecular weight of 500 or more and 180,000 or less is dextran, polyethylene glycol, or polyvinylpyrrolidone.

In a specific aspect of the blood collection container according to the present invention, the water-soluble polymer compound having a number average molecular weight of 500 or more and 180,000 or less is a water-soluble polymer compound having a number average molecular weight of 2,000 or more and 150,000 or less.

In a specific aspect of the blood collection container according to the present invention, the aqueous solution further contains trehalose.

In a specific aspect of the blood collection container according to the present invention, the aqueous solution further contains glucose, adenine, or inositol.

In a specific aspect of the blood collection container according to the present invention, the aqueous solution further contains an apoptosis inhibitor.

In a specific aspect of the blood collection container according to the present invention, the blood collection container is a blood collection container in which a predetermined amount of blood is collected, and when physiological saline in an amount equal to the predetermined amount of blood to be collected in the blood collection container is collected into the blood collection container to obtain a mixture solution in which the physiological saline and the aqueous solution are mixed, an osmotic pressure of the mixture solution is 300 mOsm/L or more.

In a specific aspect of the blood collection container according to the present invention, the aqueous solution further contains propylene glycol.

In a specific aspect of the blood collection container according to the present invention, the plasma separation material is a plasma separation composition.

In a specific aspect of the blood collection container according to the present invention, the plasma separation composition contains an organic component having fluidity at 25°C and an inorganic fine powder, the organic component contains a resin, and the inorganic fine powder contains fine powder silica.

In a specific aspect of the blood collection container according to the present invention, the fine powder silica contains hydrophilic silica.

In a specific aspect of the blood collection container according to the present invention, a content of the hydrophilic silica is 0.01 wt% or more and 2.50 wt% or less in 100 wt% of the plasma separation composition.

In a specific aspect of the blood collection container according to the present invention, the fine powder silica contains hydrophilic silica and hydrophobic silica.

In a specific aspect of the blood collection container according to the present invention, the plasma separation composition has a specific gravity at 25°C of 1.050 or more, and the inorganic fine powder contains an inorganic fine powder having a specific gravity larger than that of the fine powder silica.

In a specific aspect of the blood collection container according to the present invention, the resin contains a petroleum resin, a cyclopentadiene resin, a polyester resin, or a (meth)acrylic resin.

In a specific aspect of the blood collection container according to the present invention, the blood collection container is used for separating extracellular free nucleic acid or extracellular vesicle in blood.

According to a broad aspect of the present invention, a method for separating plasma is provided that includes the steps of collecting blood in the above-described blood collection container, and centrifuging the blood collection container in which the blood has been collected.

According to a broad aspect of the present invention, a method for separating extracellular free nucleic acid is provided that includes the steps of collecting blood in the above-described blood collection container, centrifuging the blood collection container in which the blood has been collected to separate plasma from the blood, and separating extracellular free nucleic acid from the separated plasma.

According to a broad aspect of the present invention, a method for separating extracellular vesicle is provided that includes the steps of collecting blood in the above-described blood collection container, centrifuging the blood collection container in which the blood has been collected to separate plasma from the blood, and separating extracellular vesicle from the separated plasma.

## EFFECT OF THE INVENTION

A blood collection container according to the present invention includes a blood collection container main body, a plasma separation material contained in the blood collection container main body, and an aqueous solution contained in the blood collection container main body, wherein the plasma separation material has a specific gravity at 25°C of 1.027 or more and 1.060 or less. In the blood collection container according to the present invention, the aqueous solution contains an anticoagulant and contains a water-soluble polymer compound having a number average molecular weight of 500 or more and 180,000 or less or ammonium sulfate. In the blood collection container according to the present invention, which has the above-described configuration, contamination of plasma with leukocyte-derived DNA during specimen storage can be suppressed.

**BRIEF DESCRIPTION OF DRAWINGS**

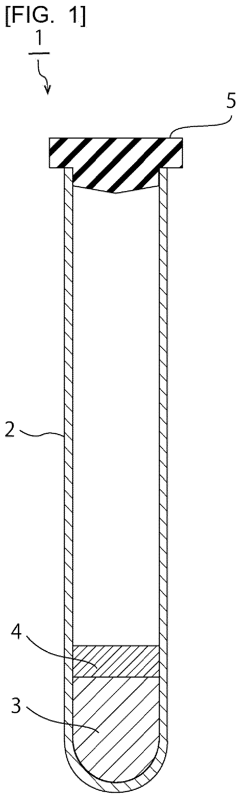

[Fig. 1] Fig. 1 is a front cross-sectional view schematically illustrating a blood collection container according to an embodiment of the present invention.

**MODE(S) FOR CARRYING OUT THE INVENTION**

Hereinafter, the present invention will be described in detail.

A blood collection container according to the present invention includes a blood collection container main body, a plasma separation material contained in the blood collection container main body, and an aqueous solution contained in the blood collection container main body, wherein the plasma separation material has a specific gravity at 25°C of 1.027 or more and 1.060 or less. In the blood collection container according to the present invention, the aqueous solution contains an anticoagulant and contains a water-soluble polymer compound having a number average molecular weight of 500 or more and 180,000 or less or ammonium sulfate.

In the blood collection container according to the present invention, which has the above-described configuration, contamination of plasma with leukocyte-derived DNA during specimen storage can be suppressed.

When blood is collected in the blood collection container according to the present invention, the blood and the aqueous solution are mixed. The present inventors have found that contamination of leukocytes into the separated plasma can be suppressed by a specific water-soluble polymer compound or ammonium sulfate contained in the aqueous solution. The present inventors also have found that contaminated leukocytes in the separated plasma can be stabilized by the specific water-soluble polymer compound or ammonium sulfate contained in the aqueous solution. Therefore, in the blood collection container according to the present invention, DNA leakage from leukocytes due to destruction over time of leukocytes is effectively suppressed, and an increase in the amount of DNA in plasma during specimen storage can be suppressed. In addition, in the blood collection container according to the present invention, since the destruction over time of leukocytes contaminated in the plasma can be effectively suppressed, the leakage of contents such as proteins from the leukocytes can also be effectively suppressed.

Hereinafter, the blood collection container according to the present invention will be described in more detail. In the present specification, "(meth) acryl" means one or both of "acryl" and "methacryl".

(Plasma separation material)

The blood collection container includes a plasma separation material contained in the blood collection container main body. As the plasma separation material, a conventionally known plasma separation material can be used. Examples of the plasma separation material include a plasma separation composition and a plasma separation jig. The plasma separation material is preferably the plasma separation composition because such a plasma separation material can be easily produced.

From the viewpoint of favorably separating plasma from blood, the plasma separation material has a specific gravity at 25°C of 1.027 or more and 1.060 or less.

The specific gravity at 25°C of the plasma separation material is preferably 1.029 or more, more preferably 1.030 or more, and further preferably 1.032 or more, as well as preferably 1.055 or less, and more preferably 1.050 or less. When the specific gravity at 25°C of the plasma separation material is at or above the lower limit and at or below the upper limit, the plasma can be more satisfactorily separated from the blood, and contamination of leukocytes and erythrocytes into the plasma can be more effectively suppressed.

The location where the plasma separation material is stored is not particularly limited as long as it is in the blood collection container main body. The plasma separation material may be disposed at a bottom position in the blood collection container main body or may be disposed on an inner wall surface of the blood collection container main body.

<Plasma separation composition>

The plasma separation composition is a composition that moves to between the plasma layer and the hemocyte layer during centrifugation to form a partition. In addition, the plasma separation composition is used for the purpose of preventing component migration between the plasma layer and the hemocyte layer after centrifugation. The plasma separation composition preferably has thixotropy. The plasma separation composition may be contained in a bottom position in the blood collection container main body or may be disposed on an inner wall surface thereof. From the viewpoint of more effectively exhibiting the effects of the present invention, it is preferable that the plasma separation composition is contained in the bottom position of the blood collection container main body.

As the plasma separation composition, a conventionally known plasma separation composition can be used.

The plasma separation composition preferably contains an organic component having fluidity at 25°C and an inorganic fine powder. Only one kind of each of the organic component having fluidity at 25°C and the inorganic fine powder may be used, and two or more kinds thereof may be used in combination.

Organic component having fluidity at 25°C:

The phrase "having fluidity at 25°C" means that the viscosity at 25°C is 500 Pa·s or less.

The viscosity of the organic component at 25°C is preferably 10 Pa·s or more, and more preferably 30 Pa·s or more, as well as preferably 200 Pa·s or less, and more preferably 100 Pa·s or less. When the viscosity is at or above the lower limit and at or below the upper limit, the fluidity of the plasma separation composition separation composition is enhanced, and the strength of the partition formed by the operation of centrifugation can be enhanced.

The viscosity of the organic component at 25°C is measured using an E-type viscometer (For example, "TVE-35" manufactured by Toki Sangyo Co., Ltd.) under the conditions of 25°C and a shear rate of $1.0 \text{ sec}^{-1}$.

Examples of the organic component include a resin, and a mixture of a resin and an organic compound such as a plasticizer. Therefore, the organic component preferably contains the resin, and more preferably contains the resin and the organic compound. When the organic component is a mixture of the resin and the organic compound, the resin or the organic compound may not have fluidity as long as the mixture (organic component) has fluidity. When the organic component is a mixture of the resin and the organic compound, the resin may be, for example, a resin that is solid at 25°C. Only one kind of each of the resin and the organic compound may be used, or two or more kinds thereof may be used in combination.

Examples of the resin include a petroleum resin, a cyclopentadiene resin, a polyester resin, a polyurethane resin, a (meth)acrylic resin, a silicone resin, an $\alpha$-olefin-fumaric acid ester copolymer, a copolymer of sebacic acid, 2,2-dimethyl-1,3-propanediol, and 1,2-propanediol, a polyether polyurethane resin, and a polyether polyester resin. One kind of the resin may be used alone, or two or more kinds of the same may be used in combination.

The resin preferably contains a petroleum resin, a cyclopentadiene resin, a polyester resin, or a (meth)acrylic resin.

Examples of commercially available products of the petroleum resin include "Regalite S5090" manufactured by Eastman Chemical Company.

Examples of the cyclopentadiene resin include a polymer of a cyclopentadiene monomer, a copolymer of a cyclopentadiene monomer and an aromatic monomer, and a dicyclopentadiene resin. The cyclopentadiene resin may be hydrogenated. The polymer of the cyclopentadiene monomer and the copolymer of the cyclopentadiene monomer and the aromatic monomer may be oligomers.

Examples of the cyclopentadiene monomer include cyclopentadiene, dicyclopentadiene, and an alkyl-substituted derivative of cyclopentadiene.

Examples of the aromatic monomer include styrene, methylstyrene, indene, and methylindene.

Examples of a commercially available product of the dicyclopentadiene resin include "SCOREZ SU500" and "SCOREZ SU90" manufactured by KOLON Industries, Inc.

Examples of the polyester resin include a polyalkylene terephthalate resin and a polyalkylene naphthalate resin. Examples of the polyalkylene terephthalate resin include polyethylene terephthalate, polybutylene terephthalate, and poly-1,4-cyclohexanedimethylene terephthalate.

Examples of the polyurethane resin include a reaction product of a polyol compound and an isocyanate compound.

Examples of the (meth)acrylic resin include a resin obtained by polymerizing at least one (meth)acrylic acid ester monomer, and a resin obtained by polymerizing at least one (meth)acrylic acid ester monomer and at least one monomer other than the (meth)acrylic acid ester monomer.

Examples of the (meth)acrylic acid ester monomer include (meth)acrylic acid alkyl ester, (meth)acrylic acid polyalkylene glycol ester, (meth)acrylic acid alkoxyalkyl ester, (meth)acrylic acid hydroxyalkyl ester, (meth)acrylic acid glycidyl ester, (meth)acrylic acid dialkylaminoalkyl ester, (meth)acrylic acid benzyl ester, (meth)acrylic acid phenoxyalkyl ester, (meth)acrylic acid cyclohexyl ester, (meth)acrylic acid isobornyl ester, and (meth)acrylic acid alkoxysilyl alkyl ester. When the (meth)acrylic acid ester monomer has an alkyl group, the number of carbon atoms of the alkyl group is preferably 1 or more and preferably 20 or less. The (meth)acrylic acid alkyl ester is preferably a (meth)acrylic acid alkyl ester having an alkyl group having 1 to 20 carbon atoms. One kind of the (meth)acrylic acid ester monomer may be used alone, or two or more kinds of the same may be used in combination.

Examples of the organic compound include a benzene polycarboxylic acid alkyl ester derivative. The organic compound is preferably a benzene polycarboxylic acid alkyl ester derivative. Therefore, the organic component is preferably a mixture of the resin and the benzene polycarboxylic acid alkyl ester derivative.

Examples of the benzene polycarboxylic acid alkyl ester derivative include a phthalic acid ester, a trimellitic acid ester, and a pyromellitic acid ester. One kind of the benzene polycarboxylic acid alkyl ester derivative may be used alone, or two or more kinds of the same may be used in combination.

Examples of the trimellitic acid ester include tri-n-octyl trimellitate, triisooctyl trimellitate, and triisodecyl trimellitate.

Examples of the pyromellitic acid ester include tetraisooctyl pyromellitate.

Examples of the commercially available product of the trimellitic acid ester include "Monocizer W700" and "Monocizer W750" manufactured by DIC Corporation, and "SANSO CIZER TOTM" and "SANSO CIZER TITM" manufactured by New Japan Chemical Co., Ltd.

Examples of the commercially available product of the pyromellitic acid ester include "Monocizer W7010" manufactured by DIC Corporation.

The benzene polycarboxylic acid alkyl ester derivative is preferably a phthalic acid ester, a trimellitic acid ester, or a pyromellitic acid ester, and more preferably a trimellitic acid ester.

The content of the organic component in 100 wt% of the plasma separation composition is preferably 80 wt% or more, more preferably 85 wt% or more, and further preferably 90 wt% or more, as well as preferably 97 wt% or less.

Inorganic fine powder:

Examples of the inorganic fine powder include fine powder silica, titanium oxide powder, calcium carbonate powder, zinc oxide powder, alumina powder, glass fine powder, talc powder, kaolin powder, bentonite powder, titania powder, and zirconium powder.

From the viewpoint of maintaining both the specific gravity and the thixotropy of the plasma separation composition in suitable ranges, the inorganic fine powder preferably contains fine powder silica. In order to obtain a plasma separation composition having a specific gravity at 25°C of 1.050 or more, the inorganic fine powder more preferably contains fine powder silica and an inorganic fine powder (second inorganic fine powder) other than the fine powder silica. However, even when the specific gravity of the plasma separation composition at 25°C is 1.050 or more, the inorganic fine powder may not contain the second inorganic fine powder. However, even when the specific gravity of the plasma separation composition at 25°C is less than 1.050, the inorganic fine powder may contain the second inorganic fine powder. For the organic fine powder, only one kind of the same may be used, or two or more kinds of the same may be used. This may apply to the fine powder silica and the second inorganic fine powder.

Examples of the fine powder silica include natural silica and synthetic silica. Examples of the synthetic silica include hydrophilic silica and hydrophobic silica. Hydrophilic silica has, for example, an action of imparting thixotropy to the plasma separation composition and adjusting the specific gravity by hydrogen bonding between hydroxyl groups on the particle surfaces. On the other hand, hydrophobic silica has a smaller thixotropy imparting effect than hydrophilic silica.

From the viewpoint of maintaining both the specific gravity and the thixotropy of the plasma separation composition in suitable ranges, the fine powder silica preferably contains hydrophilic silica, and more preferably contains hydrophilic silica and hydrophobic silica.

The second inorganic fine powder is preferably an inorganic fine powder having a specific gravity larger than that of the fine powder silica, and more preferably an inorganic fine powder having a specific gravity of 3 or more, such as a zinc oxide powder, a titanium oxide powder, or an alumina powder.

The second inorganic fine powder preferably has a specific gravity of 3 or more, more preferably 3.5 or more, and further preferably 4 or more. The specific gravity of the second inorganic fine powder is preferably as large as possible. When the specific gravity is at or above the lower limit, the specific gravity of the plasma separation composition can be effectively increased.

The average particle diameters of the inorganic fine powder, the fine powder silica, and the second inorganic fine powder are not particularly limited. The average particle diameter of the inorganic fine powder, the fine powder silica, and the second inorganic fine powder may be 1 nm or more, or 10 nm or more, as well as 500 nm or less, or 100 nm or less.

The average particle diameters of the inorganic fine powder, the fine powder silica, and the second inorganic fine powder are average diameters measured on a volume basis, and are values of median diameters (D50) corresponding to 50%. The volume average particle diameter (D50) can be measured by the laser diffraction/scattering method, the image analysis method, the Coulter method, the centrifugal sedimentation method, or the like. The volume average particle diameter (D50) is preferably determined by the measurement by the laser diffraction/scattering method or the image analysis method.

The specific surface area of the fine powder silica is not particularly limited. The specific surface area of the fine powder silica may be 20 $m^2$/g or more, or 100 $m^2$/g or more, as well as 500 $m^2$/g or less, or 300 $m^2$/g or less.

The specific surface area of the fine powder silica is measured by the BET method.

The content of the hydrophilic silica in 100 wt% of the plasma separation composition is preferably 0.01 wt% or more, more preferably 0.10 wt% or more, and further preferably 0.30 wt% or more, as well as preferably 2.50 wt% or less, and more preferably 2.00 wt% or less. When the content of the hydrophilic silica is at or above the lower limit and at or below the upper limit, both the specific gravity and the thixotropy of the plasma separation composition can be maintained in more suitable ranges.

The content of the fine powder silica in 100 wt% of the plasma separation composition is preferably 0.1 wt% or more, and more preferably 0.5 wt% or more, as well as preferably 10 wt% or less, and more preferably 7 wt% or less. When the content of the fine powder silica is at or above the lower limit and at or below the upper limit, both the specific gravity and the thixotropy of the plasma separation composition can be maintained in more suitable ranges.

The content of the second inorganic fine powder in 100 wt% of the plasma separation composition is preferably 0.01 wt% or more, and more preferably 0.1 wt% or more, as well as preferably 10 wt% or less, and more preferably 7 wt% or less. When the content of the second inorganic fine powder is at or above the lower limit and at or below the upper limit, the specific gravity of the plasma separation composition can be effectively increased.

The content of the inorganic fine powder in 100 wt% of the plasma separation composition is preferably 0.1 wt% or more, and more preferably 0.5 wt% or more, as well as preferably 10 wt% or less, and more preferably 7 wt% or less. When the content of the inorganic fine powder is at or above the lower limit and at or below the upper limit, the specific gravity of the plasma separation composition can be effectively increased.

Other components:

The plasma separation composition may contain components other than the above-described components as long as the effects of the present invention are not impaired. Examples of the other components include an organic gelling agent, a thermoplastic elastomer, a polyalkylene glycol, a silicone oil, an auxiliary solvent, an antioxidant, a colorant, water, and the like as the other components. One kind of the other components may be used alone, or two or more kinds of the same may be used in combination.

The specific gravity at 25°C of the plasma separation composition is preferably 1.027 or more, more preferably 1.029 or more, further preferably 1.030 or more, and particularly preferably 1.032 or more, as well as preferably 1.060 or less, more preferably 1.055 or less, and further preferably 1.050 or less. When the specific gravity at 25°C of the plasma separation composition is at or above the lower limit and at or below the upper limit, the plasma can be satisfactorily separated from the blood, and contamination of leukocytes into the plasma can be effectively suppressed. In addition, when the specific gravity at 25°C of the plasma separation composition is at or above the lower limit and at or below the upper limit, contamination of erythrocytes into the plasma can also be effectively suppressed. However, the specific gravity at 25°C of the plasma separation composition may be 1.050 or more, or more than 1.050.

The specific gravity at 25°C of the plasma separation composition is measured as follows: drops of the plasma separation composition are dropped one by one sequentially into saline at 25°C whose specific gravity is adjusted stepwise at intervals of 0.002, and determination is made based on float or sink of each drop in saline.

The viscosity at 25°C of the plasma separation composition is preferably 50 Pa·s or more, and more preferably 70 Pa·s or more, as well as preferably 500 Pa·s or less, and more preferably 400 Pa·s or less. When the foregoing viscosity is at or above the lower limit and at or below the upper limit, the effects of the present invention can be more effectively exhibited.

The viscosity at 25°C of the plasma separation composition is measured using an E-type viscometer (For example, "TVE-35" manufactured by Toki Sangyo Co., Ltd.) under the conditions of 25°C and a shear rate of 1.0 sec$^{-1}$.

<Plasma separation jig>

The plasma separation jig is a jig that moves to between the plasma layer and the hemocyte layer during centrifugation to form a partition. In addition, the plasma separation jig is used for the purpose of preventing component migration between the plasma layer and the hemocyte layer.

As the plasma separation jig, a conventionally known plasma separation jig can be used. Examples of the plasma separation jig include a mechanical separator (plasma separation jig) described in WO 2010/132783 A1 and the like.

Examples of the material for the plasma separation jig include elastomer.

(Aqueous solution)

The blood collection container includes aqueous solution contained in the blood collection container main body. The aqueous solution preferably contains an anticoagulant and water. The aqueous solution contains a water-soluble polymer compound having a number average molecular weight of 500 or more and 180,000 or less (hereinafter, it may be referred to as water-soluble polymer compound (X)) or ammonium sulfate. The solute contained in the aqueous solution contains an anticoagulant. The solute contained in the aqueous solution contains the water-soluble polymer compound (X)

or ammonium sulfate. The aqueous solution may contain the water-soluble polymer compound (X) and ammonium sulfate.

<Anticoagulant>

The aqueous solution contains an anticoagulant. As the anticoagulant, a conventionally known anticoagulant can be used. One kind of the anticoagulant may be used alone, or two or more kinds of the same may be used in combination.

Examples of the anticoagulant include heparin, metal salts of heparin, ethylenediaminetetraacetic acid (EDTA), metal salts of EDTA, citric acid, and sodium citrate.

From the viewpoint of favorably exhibiting anticoagulation performance, the anticoagulant is preferably EDTA, a metal salt of EDTA, heparin, a metal salt of heparin, or sodium citrate.

The concentration of the anticoagulant in the aqueous solution is preferably 2 mM or more, more preferably 5 mM or more, and further preferably 10 mM or more, as well as preferably 2000 mM or less, more preferably 1000 mM or less, still more preferably 500 mM or less, further preferably 250 mM or less, still further preferably 100 mM or less, and particularly preferably 50 mM or less. When the foregoing concentration of the anticoagulant is at or above the lower limit and at or below the upper limit, good anticoagulant performance can be achieved.

<Water-soluble polymer compound having number average molecular weight of 500 or more and 180,000 or less (water-soluble polymer compound (X))>

The aqueous solution preferably contains a water-soluble polymer compound having a number average molecular weight of 500 or more and 180,000 or less (water-soluble polymer compound (X)). The reason why contamination of leukocytes into the separated plasma can be suppressed by using the water-soluble polymer compound (X) is presumed to be that the water-soluble polymer compound (X) keeps good viscosity of blood, so that movement of leukocytes during centrifugation is performed more smoothly, but the present invention is not limited thereto. The reason why leakage of DNA from leukocytes contaminated in plasma is suppressed by using the water-soluble polymer compound (X) is presumed to be that the water-soluble polymer compound (X) effectively protects the cell membrane of leukocytes to stabilize leukocytes, but is not limited thereto. Incidentally, the water solubility in the water-soluble polymer compound (X) means that 0.1 g or more of the water-soluble polymer compound (X) is dissolved in 100 g of water at 25°C. One kind of the water-soluble polymer compound (X) may be used alone, or two or more kinds thereof may be used in combination.

From the viewpoint of exerting the effects of the present invention, the number average molecular weight of the water-soluble polymer compound (X) is 500 or more and 180,000 or less.

The number average molecular weight of the water-soluble polymer compound (X) is preferably 1,000 or more, more preferably 1,500 or more, further preferably 2,000 or more, and particularly preferably 2,500 or more, as well as preferably 170,000 or less, more preferably 150,000 or less, further preferably 120,000 or less, and particularly preferably 100,000 or less. When the number average molecular weight of the water-soluble polymer compound (X) is at or above the lower limit and at or below the upper limit, contamination of leukocytes into the separated plasma can be further suppressed, and leukocytes can be further stabilized, whereby the effects of the present invention can be more effectively exhibited.

The number average molecular weight of the water-soluble polymer compound (X) is a number average molecular weight in terms of standard polyethylene glycol measured by gel permeation chromatography (GPC).

Examples of the water-soluble polymer compound (X) include dextran, polyethylene glycol, polyvinylpyrrolidone, polyvinyl alcohol, methyl cellulose, hydroxyethyl cellulose, and corn starch.

The water-soluble polymer compound (X) is preferably dextran, polyethylene glycol, or polyvinylpyrrolidone. In this case, the leukocytes can be further stabilized, whereby the effects of the present invention can be more effectively exhibited.

Each content of the water-soluble polymer compound (X) in 100 wt% of the aqueous solution is preferably 0.1 wt% or more, more preferably 0.5 wt% or more, and further preferably 1 wt% or more, as well as preferably 15 wt% or less, more preferably 10 wt% or less, and further preferably 7 wt% or less. When each content of the water-soluble polymer compound (X) is at or above the lower limit and at or below the upper limit, the leukocytes can be further stabilized, whereby the effects of the present invention can be more effectively exhibited. When each content of the water-soluble polymer compound (X) is at or above the lower limit, the osmotic pressure of the mixture solution obtained by mixing the collected blood and the aqueous solution can be effectively increased, so that the amount of leukocytes contaminated in the plasma can be further reduced. It should be noted that the "each content of the water-soluble polymer compound (X)" means the content of the water-soluble polymer compound (X) when the aqueous solution contains one type of the water-soluble polymer compound (X), and means each of the contents of the water-soluble polymer compounds (X) when the aqueous solution contains two or more types of the water-soluble polymer compounds (X).

The content of the water-soluble polymer compound (X) in 100 wt% of the aqueous solution is preferably 0.1 wt% or more, more preferably 0.5 wt% or more, and further preferably 1 wt% or more, as well as preferably 20 wt% or less, more preferably 15 wt% or less, and further preferably 10 wt% or less. When the content of the water-soluble polymer compound (X) is at or above the lower limit and at or below the upper limit, the leukocytes can be further stabilized, whereby the effects of the present invention can be more effectively exhibited. When the content of the water-soluble polymer compound (X) is at or above the lower limit, the osmotic pressure of the mixture solution obtained by mixing the collected blood and the aqueous solution can be effectively increased, so that the amount of leukocytes contaminated in the plasma can be further reduced.

<Ammonium sulfate>

The aqueous solution preferably contains ammonium sulfate. The present inventors have found that contamination of leukocytes into the separated plasma can be further suppressed by using ammonium sulfate, and leakage of DNA from the leukocytes contaminated in the plasma is suppressed. When the aqueous solution contains the water-soluble polymer compound (X) and ammonium sulfate, the effects of the present invention are more effectively exhibited.

The content of the ammonium sulfate in 100 wt% of the aqueous solution is preferably 0.5 wt% or more, more preferably 1 wt% or more, and further preferably 4 wt% or more, as well as preferably 20 wt% or less, more preferably 15 wt% or less, and further preferably 10 wt% or less. When the content of the ammonium sulfate is at or above the lower limit and at or below the upper limit, contamination of leukocytes into the separated plasma can be further suppressed, and leukocytes can be further stabilized, whereby the effects of the present invention can be more effectively exhibited. When the content of the ammonium sulfate is at or above the lower limit, the osmotic pressure of the mixture solution obtained by mixing the collected blood and the aqueous solution can be effectively increased, so that the amount of leukocytes contaminated in the plasma can be further reduced.

<Trehalose>

From the viewpoint of more effectively exhibiting the effects of the present invention, the aqueous solution preferably contains trehalose. The solute contained in the aqueous solution preferably contains trehalose. By using trehalose, the cell membranes of leukocytes are effectively protected, and leukocytes can be stabilized, which results in that leakage of DNA from leukocytes contaminated in plasma can be further suppressed.

The content of trehalose in 100 wt% of the aqueous solution is preferably 0.5 wt% or more, more preferably 1 wt% or more, and further preferably 2 wt% or more, as well as preferably 10 wt% or less, more preferably 7 wt% or less, and further preferably 5 wt% or less. When the content of the trehalose is at or above the lower limit and at or below the upper limit, the leukocytes can be further stabilized, whereby the effects of the present invention can be more effectively exhibited. When the content of the trehalose is at or above the lower limit, the osmotic pressure of the mixture solution obtained by mixing the collected blood and the aqueous solution can be effectively increased, so that the amount of leukocytes contaminated in the plasma can be further reduced.

<Glucose, adenine, inositol>

From the viewpoint of more effectively exhibiting the effects of the present invention, the aqueous solution preferably contains glucose, adenine, or inositol. The solute contained in the aqueous solution preferably contains glucose, adenine, or inositol. In this case, the aqueous solution may contain only one of glucose, adenine, and inositol, or may contain two or more of these. By using these compounds, nutrients are supplied to leukocytes, which makes it possible to increase the survival rate of leukocytes, and as a result, leakage of DNA from leukocytes contaminated in plasma can be further suppressed.

When the aqueous solution contains glucose, the content of glucose in 100 wt% of the aqueous solution is preferably 0.1 wt% or more, and more preferably 0.3 wt% or more, as well as preferably 10 wt% or less, and more preferably 5 wt% or less. When the content of the glucose is at or above the lower limit and at or below the upper limit, the effects of the present invention can be more effectively exhibited. When the content of the glucose is at or above the lower limit, the osmotic pressure of the mixture solution obtained by mixing the collected blood and the aqueous solution can be effectively increased, so that the amount of leukocytes contaminated in the plasma can be further reduced.

When the aqueous solution contains adenine, the content of adenine in 100 wt% of the aqueous solution is preferably 0.01 wt% or more, and more preferably 0.05 wt% or more, as well as preferably 2 wt% or less, and more preferably 1 wt% or less. When the content of the adenine neutralizing agent is at or above the lower limit and at or below the upper limit, the effects of the present invention can be more effectively exhibited. When the content of the adenine is at or above the lower limit, the osmotic pressure of the mixture solution obtained by mixing the collected blood and the aqueous solution can be effectively increased, so that the amount of leukocytes contaminated in the plasma can be further reduced.

When the aqueous solution contains inositol, the content of inositol in 100 wt% of the aqueous solution is preferably 0.01 wt% or more, and more preferably 0.05 wt% or more, as well as preferably 2 wt% or less, and more preferably 1 wt% or less. When the content of the inositol neutralizing agent is at or above the lower limit and at or below the upper limit, the effects of the present invention can be more effectively exhibited. When the content of the inositol is at or above the lower limit, the osmotic pressure of the mixture solution obtained by mixing the collected blood and the aqueous solution can be effectively increased, so that the amount of leukocytes contaminated in the plasma can be further reduced.

<Apoptosis Inhibitor>

From the viewpoint of more effectively exhibiting the effects of the present invention, the aqueous solution preferably contains an apoptosis inhibitor. The solute contained in the aqueous solution preferably contains an apoptosis inhibitor. By using an apoptosis inhibitor, apoptosis of leukocytes during specimen storage are effectively suppressed, which results in that leakage of DNA from leukocytes contaminated in plasma can be further suppressed. One kind of the apoptosis inhibitor may be used alone, or two or more kinds of the same may be used in combination.

Examples of the apoptosis inhibitor include Q-VD-Oph and Z-VAD-FMK.

From the viewpoint of more effectively exhibiting the effects of the present invention, the apoptosis inhibitor is preferably a caspase inhibitor, and more preferably Q-VD-Oph. The aqueous solution preferably contains a caspase inhibitor, and more preferably contains Q-VD-Oph.

The concentration of the apoptosis inhibitor in the aqueous solution is preferably 1 nM or more, and more preferably 20 nM or more, as well as preferably 10 $\mu$M or less, and more preferably 5 $\mu$M or less. When the concentration of the apoptosis inhibitor is at or above the lower limit and at or below the upper limit, the effects of the present invention can be more effectively exhibited.

<Propylene glycol>

From the viewpoint of more effectively exhibiting the effects of the present invention, the aqueous solution preferably contains propylene glycol. The solute contained in the aqueous solution preferably contains propylene glycol.

The content of propylene glycol in 100 wt% of the aqueous solution is preferably 1 wt% or more, and more preferably 5 wt% or more, as well as preferably 50 wt% or less, and more preferably 30 wt% or less.

<Other components>

The aqueous solution may contain components other than the above-described components (anticoagulant, water-soluble polymer compound (X), ammonium sulfate, trehalose, glucose, adenine, inositol, apoptosis inhibitor, and propylene glycol) .

Examples of the other components include inorganic salts, saccharides, and sugar alcohols. One kind of the other components may be used alone, or two or more kinds of the same may be used in combination.

Examples of the inorganic salt include sodium salts such as sodium chloride and sodium hydrogen phosphate, and potassium salts such as potassium chloride and potassium hydrogen carbonate.

Examples of the saccharide include dihydroxyacetone, fructose, galactose, sucrose, maltose, lactulose, and polysaccharides not corresponding to the water-soluble polymer compound (X).

Examples of the sugar alcohol include D-mannitol and D-sorbitol.

The amount of the aqueous solution contained in the blood collection container main body is appropriately changed according to the size of the blood collection container main body, the amount of collected blood, and the like. The amount of the aqueous solution contained in the blood collection container main body is preferably 0.1 mL or more, more preferably 0.5 mL or more, and further preferably 0.7 mL or more, as well as preferably 5 mL or less, more preferably 3 mL or less, and further preferably 2.5 mL or less. When the amount of the aqueous solution is at or above the lower limit and at or below the upper limit, blood is not excessively diluted, and the effects of the present invention can be more effectively exhibited.

(Blood collection container main body)

The shape of the blood collection container main body is not particularly limited. The blood collection container main body is preferably a bottomed tubular container.

The material of the blood collection container main body is not particularly limited. Examples of the material of the blood collection container main body include thermoplastic resins such as polyethylene, polypropylene, polystyrene, polyethylene terephthalate, polymethyl methacrylate, and polyacrylonitrile; thermosetting resins such as unsaturated polyester resins, epoxy resins, and epoxy-acrylate resins; modified natural resins such as cellulose acetate, cellulose propionate, ethyl cellulose, and ethyl chitin; silicate glasses such as soda lime glass, phosphosilicate glass, and borosilicate glass; and glasses such as quartz glass. As the material of the blood collection container main body, one kind may be used alone, or two or more kinds may be used in combination.

(Plug)

The blood collection container preferably includes a plug. The plug is preferably attached to the opening of the blood collection container main body. As the plug, a conventionally known plug can be used. The plug is preferably formed of a such material in such a shape that the plug can be airtightly and liquid-tightly attached to the opening of the blood collection container main body. The plug is preferably configured such that a blood sampling needle can be inserted therethrough.

Examples of the plug include a plug having a shape fitted to the opening of the blood collection container main body, a sheet-like seal plug, and the like.

The plug may include a plug main body such as a rubber plug and a cap member made of plastic or the like. In this case, it is possible to suppress the risk that the blood comes into contact with a human body when the plug is pulled out from the opening of the blood collection container main body after the blood collection.

Examples of the material for the plug (or the plug main body) include synthetic resins, elastomers, rubbers, and metal foils. Examples of the rubber include butyl rubber and halogenated butyl rubber. Examples of the metal foil include an aluminum foil. From the viewpoint of enhancing the sealing property, the material for the plug is preferably butyl rubber. The plug (or the plug main body) is preferably a butyl rubber plug.

(Other details of blood collection container)

The blood collection container is a blood collection container into which a predetermined amount of blood is collected. The predetermined amount of blood is appropriately changed depending on the size, internal pressure, and the like of the blood collection container. The predetermined amount of blood may be 1 mL or more, 2 mL or more, and 4 mL or more, as well as 12 mL or less, 11 mL or less, and 10 mL or less.

An amount of physiological saline equal to the predetermined amount of blood to be collected in the blood collection container is collected in the blood collection container to obtain a mixture solution in which the physiological saline and the aqueous solution are mixed. For example, in the blood collection container in which 5 mL of blood is collected, 5 mL of physiological saline is collected in the blood collection container, and the physiological saline and the aqueous solution are mixed by inversion mixing or the like, whereby a mixture solution is obtained. The osmotic pressure of the mixture solution in which the physiological saline and the aqueous solution are mixed is preferably 300 mOsm/L or more, more preferably 330 mOsm/L or more, and further preferably 350 mOsm/L or more, as well as preferably 1300 mOsm/L or less, more preferably 1000 mOsm/L or less, and further preferably 800 mOsm/L or less. When the osmotic pressure of the mixture solution is at or above the lower limit, the blood and the aqueous solution are mixed when blood is collected in the blood collection container, and the osmotic pressure of the blood increases. Therefore, the moisture in the leukocytes and the moisture in the erythrocytes move to the outside of the hemocytes, and the specific gravities of the leukocytes and the erythrocytes increase. Centrifuging the blood collection container causes the leukocytes and erythrocytes having greater specific gravities to move satisfactorily downward to below the plasma separation material. As a result, contamination of leukocytes and erythrocytes into plasma can be effectively suppressed. When the osmotic pressure of the mixture solution is at or above the lower limit and at or below the upper limit, excessive stress on the leukocytes is suppressed, which further stabilizes the leukocytes. Therefore, the effects of the present invention can be more effectively exhibited.

The osmotic pressure of the mixture solution is measured by the freezing point depressing method using an osmosis meter (for example, "OM-6060" manufactured by ARKRAY Inc.).

In the blood collection container, preferably 3 mL or more, more preferably 4 mL or more, and preferably 11 mL or less, more preferably 10 mL or less of blood is collected with respect to 1 mL of the aqueous solution contained therein. In this case, blood is not excessively diluted, and the effects of the present invention can be more effectively exhibited.

The blood collection container is preferably a blood collection tube. The blood collection container main body is preferably a blood collection tube main body.

The blood collection container is preferably used for separating plasma from blood. In addition, the blood collection container is preferably used for separating extracellular free nucleic acid or extracellular vesicle in blood, and is preferably used for isolating extracellular free nucleic acid or extracellular vesicle in blood.

The blood collection container can be manufactured, for example, as follows.

An anticoagulant, as well as a water-soluble polymer compound (X) or ammonium sulfate, are dissolved in water to obtain an aqueous solution. If necessary, components other than these (trehalose, glucose, adenine, inositol, an apoptosis inhibitor, propylene glycol, the other components described above, and the like) are also dissolved in water to obtain an aqueous solution. The resulting aqueous solution is added into the blood collection container main body. Before or after the aqueous solution is added, the plasma separation material is contained in the blood collection container main body.

Fig. 1 is a front cross-sectional view schematically illustrating a blood collection container according to an embodiment of the present invention.

A blood collection container 1 shown in Fig. 1 includes a blood collection container main body 2, a plasma separation composition 3, an aqueous solution 4, and a plug 5. The blood collection container main body 2 has an opening at one end thereof and a closed bottom at the other end thereof. The plasma separation composition 3 is contained in a bottom position of the blood collection container main body 2. The aqueous solution 4 contains an anticoagulant, as well as a water-soluble polymer compound (X) or ammonium sulfate. The plug 5 is inserted into the opening of the blood collection container main body 2.

The aqueous solution 4 is disposed on a surface of the plasma separation composition 3, and more specifically, on a top surface (a surface on one end side) of the plasma separation composition 3. The aqueous solution 4 is disposed on a surface of the plasma separation composition 3 when the blood collection container 1 is in an upright state.

In the blood collection container according to the present invention, the plasma separation composition may be disposed on a side wall surface of the blood collection container main body, and the aqueous solution may be disposed in a bottom position in the blood collection container main body when the blood collection container is in an upright state. In addition, the plasma separation jig may be used instead of the plasma separation composition.

The blood collection container may have an internal pressure that is not particularly limited. The blood collection container can be used as a vacuum blood collection tube that is sealed by the plug after the inside is evacuated. When the blood collection container is a vacuum blood collection tube, it is possible to easily collect a certain amount of blood regardless of the technical difference of the blood collector.

From the viewpoint of preventing bacterial infection, the inside of the blood collection container is preferably sterilized in accordance with the standards described in ISO or JIS.

(Method for separating plasma)

By using the blood collection container, plasma can be separated from blood. The method for separating plasma according to the present invention includes the steps of: collecting blood in the above-described blood collection container; and centrifuging the blood collection container in which the blood has been collected.

The method for separating plasma according to the present invention preferably includes a step of mixing the collected blood with the aqueous solution between the step of collecting the blood and the step of centrifuging. Examples of the method for mixing the collected blood with the aqueous solution include inversion mixing.

The centrifugation conditions in the centrifugation step are not particularly limited as long as a partition can be formed with the plasma separation material to separate plasma and hemocytes. Examples of the centrifugation conditions include a condition of performing centrifugation at 400 G or more and 4000 G or less for 10 minutes or more and 120 minutes or less.

(Method for separating extracellular free nucleic acid and method for separating extracellular vesicle)

The method for separating extracellular free nucleic acid according to the present invention includes the steps of: collecting blood in the above-described blood collection container; centrifuging the blood collection container in which the blood has been collected to separate plasma from the blood; and separating extracellular free nucleic acid from the separated plasma.

The method for separating extracellular vesicles according to the present invention includes the steps of: collecting blood in the above-described blood collection container; centrifuging the blood collection container in which the blood has been collected to separate plasma from the blood; and separating extracellular vesicles from the separated plasma.

The method for separating extracellular free nucleic acid and the method for separating extracellular vesicle according to the present invention preferably includes a step of mixing the collected blood with the aqueous solution between the step of collecting the blood and the step of centrifuging. Examples of the method for mixing the collected blood with the aqueous solution include inversion mixing.

The centrifugation conditions in the centrifugation step are not particularly limited as long as a partition can be formed with the plasma separation material to separate plasma and hemocytes. Examples of the centrifugation conditions include a condition of performing centrifugation at 400 G or more and 4000 G or less for 10 minutes or more and 120 minutes or less.

In the step of separating extracellular free nucleic acid, the extracellular free nucleic acid can be separated from the plasma using a conventionally known method. Examples of the extracellular free nucleic acid include cell free DNA (cfDNA) and cell free RNA (cfRNA). Examples of the method for separating extracellular free nucleic acid from plasma include a method using a commercially available nucleic acid purification kit. By using a commercially available nucleic acid purification kit, extracellular free nucleic acid can be easily separated from plasma. Examples of commercially available products of the nucleic acid purification kit include QIAamp Circulating Nucleic Acid Kit (manufactured by QIAGEN), QIAamp MinElute ccfDNA Kits (manufactured by QIAGEN), and MagMAX Cell-Free DNA Isolation Kit (manufactured by Applied Biosystems).

In the step of separating extracellular vesicles, extracellular vesicles can be separated from plasma using a conventionally known method.

Hereinafter, the present invention is described in more detail with reference to Examples. The present invention is not limited only to the following examples.

The following materials were prepared as materials for a plasma separation composition.

(Materials of organic component having fluidity at 25°C)

(Meth)acrylic resin 1:

(Meth)acrylic acid ester-based copolymer ((meth)acrylic resin 1) having fluidity at 25°C was obtained by radical-polymerizing 2-ethylhexyl acrylate and butyl acrylate in the presence of an azo-based polymerization initiator by a solution polymerization method. The (meth)acrylic resin 1 had a specific gravity at 25°C of 1.034.

(Meth)acrylic resin 2:

(Meth)acrylic acid ester-based copolymer ((meth)acrylic resin 2) having fluidity at 25°C was obtained by a method that was same as the method for producing the (meth)acrylic resin 1 except that the blending ratio of 2-ethylhexyl acrylate and butyl acrylate was changed. The (meth)acrylic resin 2 had a specific gravity at 25°C of 1.015.

Other resins:

Petroleum resin ("Regalite S5090" manufactured by Eastman Chemical Company)
Dicyclopentadiene resin 1 ("SCOREZ SU500" manufactured by KOLON Industries, Inc.)
Dicyclopentadiene resin 2 ("SCOREZ SU90" manufactured by KOLON Industries, Inc.)

Organic compound:

Trimellitic acid ester (benzene polycarboxylic acid alkyl ester derivative, "Monocizer W700" manufactured by DIC Corporation)

(Inorganic fine powder)

Hydrophilic silica (fine powder silica, "200CF" manufactured by Nippon Aerosil Co., Ltd.)
Hydrophobic silica (fine powder silica, "R974" manufactured by Nippon Aerosil Co., Ltd.)
Titanium oxide powder ("A-100" manufactured by Ishihara Sangyo Kaisha, Ltd.)

(Other components)

Silicone oil ("SF8410" manufactured by Toray Dow Corning, Co., Ltd.)
Organic gelling agent ("Gelall D" manufactured by New Japan Chemical Co., Ltd.)
1-methyl-2-pyrrolidone (auxiliary solvent)

Production of plasma separation compositions A, B, and D:

Plasma separation compositions A, B, and D were produced by mixing an organic component having fluidity at 25°C, an inorganic fine powder, and other components at blending ratios shown in Table 1.

Production of plasma separation composition C:

Materials of an organic component having fluidity at 25°C described in Table 1 were blended, heated and dissolved at 130°C, and mixed to produce an organic component having fluidity at 25°C. A plasma separation composition C was then produced by mixing an organic component having fluidity at 25°C, an inorganic fine powder, and other components at a blending ratio shown in Table 1.

[Table 1]

| | | | | Plasma separation composition A | Plasma separation composition B | Plasma separation composition C | Plasma separation composition D |
|---|---|---|---|---|---|---|---|
| Organic component having fluidity at 25°C | (Meth)acrylic resin 1 | | wt% | 97.85 | 97.05 | – | – |
| | (Meth)acrylic resin 2 | | wt% | – | – | – | 96.70 |
| | Petroleum resin | | wt% | – | – | 7.83 | – |
| | Dicyclopentadiene resin 1 | | wt% | – | – | 11.33 | – |
| | Dicyclopentadiene resin 2 | | wt% | – | – | 13.03 | – |
| | Trimellitic acid ester | | wt% | – | – | 64.90 | – |
| Inorganic fine powder | Fine powder silica | Hydrophilic silica | wt% | 1.00 | 1.00 | 0.70 | 2.00 |
| | | Hydrophobic silica | wt% | 0.85 | 0.85 | 1.80 | 1.00 |
| | Titanium oxide powder | | wt% | – | 0.80 | – | – |
| Other components | Silicone oil | | wt% | 0.30 | 0.30 | – | 0.30 |
| | Organic gelling agent | | wt% | – | – | 0.06 | – |
| | 1-methyl-2-pyrrolidone | | wt% | – | – | 0.34 | – |
| Total | | | wt% | 100 | 100 | 100 | 100 |

Drops of the plasma separation composition were dropped one by one sequentially into saline at 25 °C whose specific gravity was adjusted stepwise at intervals of 0.002, and the specific gravity was determined based on float or sink of each drop in saline. The specific gravities at 25°C of the obtained plasma separation compositions are shown in Tables 2 to 5.

The following were prepared as solutes of the aqueous solution.

(Anticoagulant)

Ethylenediaminetetraacetic acid dipotassium salt dihydrate (EDTA2K•2H$_2$O)

(Water-soluble polymer compound (X))

Dextran (number average molecular weight: 40,000)
Dextran (number average molecular weight: 70,000)
Polyethylene glycol (number average molecular weight: 3,000)
Polyethylene glycol (number average molecular weight: 20,000)
Polyvinylpyrrolidone (number average molecular weight: 10,000)
Polyvinylpyrrolidone (number average molecular weight: 58,000)

(Water-soluble polymer compound not corresponding to water-soluble polymer compound (X))

Dextran (number average molecular weight: 200,000)
Polyethylene glycol (number average molecular weight: 500,000)
Ammonium sulfate
Trehalose
Glucose
Adenine

Myo-inositol
Apoptosis Inhibitor (Q-VD-Oph)
Propylene glycol

(Other components)

Sodium chloride (osmotic pressure regulator)

The following was prepared as a blood collection container main body.

A 100 mm-long bottomed tube made of polyethylene terephthalate (PET) with an opening having an inner diameter of 14 mm

(Example 1)

The components shown in Table 2 were dissolved in water to obtain an aqueous solution. The types and concentrations of the components in the obtained aqueous solution are shown in Table 2.

The plasma separation composition A (1.2 g) was contained in a bottom position of the blood collection container main body. In addition, 1 mL of the aqueous solution was added onto the surface of the plasma separation composition A. The inside of the blood collection container was depressurized so that amount of the collected blood was 8 mL, and was sealed with a butyl rubber plug. In this way, a blood collection container was produced.

(Examples 2 to 12 and Comparative Examples 2 and 3)

The types of the plasma separation composition and the formulations of the aqueous solution were changed as shown in Tables 2 to 5. A blood collection container was prepared in the same manner as in Example 1 except for these.

(Comparative Example 1)

The anticoagulant (24 parts by weight) was dissolved in 76 parts by weight of water to obtain a mixture solution. The plasma separation composition A (1.2 g) was contained in a bottom position of the blood collection container main body. In addition, 60 mg of the obtained mixture solution was applied to the inner wall surface of the blood collection container main body and was dried. The inside of the blood collection container was depressurized so that amount of the collected blood was 8 mL, and was sealed with a butyl rubber plug. In this way, a blood collection container was produced.

(Evaluation)

(1) Osmotic pressure of mixture solution

In the obtained blood collection container, 8 mL of physiological saline was collected. The physiological saline was collected and then mixed by inversion mixing, and the physiological saline was mixed with the aqueous solution (Examples 1 to 12 and Comparative Examples 2 and 3) or the water-soluble component (Comparative Example 1) contained in the blood collection container to obtain a mixture solution. The osmotic pressure of the obtained mixture solution was measured by the freezing point depressing method using an osmosis meter ("OM-6060" manufactured by ARKRAY Inc.).

(2) Leukocyte residual amount

Blood was prepared for three persons, and the following operations were performed for each of them. One blood collection container was prepared for each, and 8 mL of blood was collected in each blood collection container. After blood was collected, the blood was mixed by inversion mixing with the aqueous solution (Examples 1 to 12 and Comparative Examples 2 and 3) or the water-soluble component (Comparative Example 1) contained in the blood collection container. The blood collection container was then centrifuged at 1500 G for 15 minutes. After centrifugation, plasma was located above a partition formed with the plasma separation composition. The plasma located above the partition was stirred by pipetting so that hemocytes deposited and remaining on the partition were suspended, and then the plasma was recovered. The recovered plasma was analyzed using a multi-item automatic hemocyte analyzer ("XE5000" manufactured by Sysmex Corporation) to count the number of leukocytes in the plasma. In addition, for the prepared blood (whole blood sample), the number of leukocytes in the whole blood sample was measured in the same manner. The number of leukocytes was an average value of results obtained by evaluating the prepared blood of the three persons. The leukocyte residual rate was calculated by the following equation.

Leukocyte residual rate (%) = (Number of leukocytes (cells) contained in separated plasma)/(Number of leukocytes (cells) contained in whole blood sample)×100

<Criteria for determination of leukocyte residual amount>

○: Leukocyte residual rate is less than 10%.
×: Leukocyte residual rate is 10% or more.

(3) Amount of cfDNA recovered

Blood was prepared for three persons, and the following operations were performed for each of them. Two blood collection containers were prepared, and 8 mL of blood was collected in each of the two blood collection containers. After blood was collected, the blood was mixed by inversion mixing with the aqueous solution (Examples 1 to 12 and Comparative Examples 2 and 3) or the water-soluble component (Comparative Example 1) contained in the blood collection container. The blood collection container was then centrifuged at 1500 G for 15 minutes. After centrifugation, plasma was located above a partition formed with the plasma separation composition.

For one of the two blood collection containers after plasma separation, plasma was recovered from the blood collection container on the day of blood collection. For the remaining one of the two blood collection containers after the plasma separation, the blood collection container from which the plasma had been separated was stored at room temperature (25°C) for 7 days, and then the plasma was recovered from the blood collection container.

DNA contained in the recovered plasma was purified using cfDNA purification kit ("QIAamp Circulating Nucleic Acid Kit" manufactured by QIAGEN). The DNA purification operation was performed on the day when plasma was recovered from the blood collection container.

The DNA concentration in the extract after purification was measured using Qubit dsDNA HS Assay kit (manufactured by Invitrogen). Next, the cfDNA concentration (the content of cfDNA contained per 1 mL of plasma) was calculated according to the following formula.

$$\text{cfDNA concentration (ng/plasma 1 mL)} = [A] \times [B]/[C]$$

[A] : Measured value of DNA concentration in extract after purification (ng/mL)
[B]: Total amount of extract after purification (mL)
[C]: Amount of plasma used for DNA purification (mL)

The average value of the concentrations of cfDNA recovered from the plasma on the day of blood collection was defined as "cfDNA concentration (collection day)", and the average value of the concentration of cfDNA recovered from the plasma after storage for 7 days was defined as "cfDNA concentration (storage for 7 days) ". In addition, the difference between the cfDNA concentration (storage for 7 days) and the cfDNA concentration (collection day) was defined as an "increase in the cfDNA concentration". The average value of the concentrations of cfDNA refers to an average value of results obtained using blood of three persons.

The amount of cfDNA recovered was determined according to the following criteria. Incidentally, the increase in the cfDNA concentration increases as the leukocytes contaminated in the plasma are destroyed during storage. Therefore, as the increase in cfDNA concentration is smaller, leakage of DNA from leukocytes contaminated in plasma is further suppressed, and contamination of leukocyte-derived DNA into the plasma during specimen storage is suppressed.

<Criteria for determination of amount of cfDNA recovered>

○○: Increase in cfDNA concentration is less than 10 ng/plasma 1 mL
○: Increase in cfDNA concentration is 10 ng/plasma 1 mL or more and less than 70 ng/plasma 1 mL.
×: Increase in cfDNA concentration is 70 ng/plasma 1 mL or more.

The compositions and the results are shown in Tables 2 to 5. In the tables, the concentration of the anticoagulant means not the concentration of EDTA2K 2H$_2$O but the concentration of EDTA2K.

[Table 2]

[Table 2]

| | | | | Example 1 | Example 2 | Example 3 | Example 4 |
|---|---|---|---|---|---|---|---|
| Aqueous solution | Anticoagulant | EDTA2K·2H$_2$O | mM | 20 | 20 | 20 | 20 |
| | Water-soluble polymer compound (X) | Dextran (Mn: 40,000) | wt% | – | – | – | – |
| | | Dextran (Mn: 70,000) | wt% | 5.0 | 5.0 | 5.0 | – |
| | | Polyethylene glycol (Mn: 3,000) | wt% | 3.0 | – | 7.0 | 1.0 |
| | | Polyethylene glycol (Mn: 20,000) | wt% | – | – | – | – |
| | | Polyvinylpyrrolidone (Mn: 10,000) | wt% | – | 1.0 | – | – |
| | | Polyvinylpyrrolidone (Mn: 58,000) | wt% | – | – | – | – |
| | Water-soluble polymer compound not corresponding to water-soluble polymer compound (X) | Dextran (Mn: 200,000) | wt% | – | – | – | – |
| | | Polyethylene glycol (Mn: 500,000) | wt% | – | – | – | – |
| | Ammonium sulfate | | wt% | – | – | – | – |
| | Osmotic pressure regulator | Sodium chloride | wt% | 2.6 | 1.4 | 2.0 | 4.9 |
| | Trehalose | | wt% | – | – | 5.0 | – |
| | Glucose | | wt% | – | – | – | – |
| | Adenine | | wt% | – | – | – | – |
| | myo-inositol | | wt% | – | – | – | – |
| | Apoptosis Inhibitor | Q-VD-Oph | µM | – | – | – | – |
| | Propylene glycol | | wt% | – | – | – | 20 |
| Amount of aqueous solution contained in blood collection container | | | mL | 1 | 1 | 1 | 1 |
| Plasma separation composition | Type | | – | A | C | A | C |
| | Specific gravity at 25°C | | – | 1.045 | 1.035 | 1.045 | 1.035 |
| Amount of blood collected | | | mL | 8 | 8 | 8 | 8 |
| Evaluation | Osmotic pressure of mixture solution | | mOsm/L | 353 | 310 | 348 | 726 |
| | Leukocyte residual amount | | % | 4 | 2 | 3 | 1 |
| | | | Determination | O | O | O | O |
| | Amount of cfDNA recovered | cfDNA concentration (collection day) | ng/plasma 1 mL | 19 | 19 | 15 | 20 |
| | | cfDNA concentration (storage for 7 days) | ng/plasma 1 mL | 50 | 37 | 30 | 27 |
| | | Increase in cfDNA concentration | ng/plasma 1 mL | 31 | 18 | 15 | 7 |
| | | | Determination | O | O | O | OO |

18

[Table 3]

| | | | | Example 5 | Example 6 | Example 7 | Example 8 |
|---|---|---|---|---|---|---|---|
| Aqueous solution | Anticoagulant | EDTA2K·2H₂O | mM | 20 | 40 | 20 | 10 |
| | Water-soluble polymer compound (X) | Dextran (Mn: 40,000) | wt% | – | 5.0 | – | 5.0 |
| | | Dextran (Mn: 70,000) | wt% | – | – | – | – |
| | | Polyethylene glycol (Mn: 3,000) | wt% | – | – | – | – |
| | | Polyethylene glycol (Mn: 20,000) | wt% | – | – | 1.0 | 7.0 |
| | | Polyvinylpyrrolidone (Mn: 10,000) | wt% | 5.0 | – | – | – |
| | | Polyvinylpyrrolidone (Mn: 58,000) | wt% | – | 1.0 | – | – |
| | Water-soluble polymer compound not corresponding to water-soluble polymer compound (X) | Dextran (Mn: 200,000) | wt% | – | – | – | – |
| | | Polyethylene glycol (Mn: 500,000) | wt% | – | – | – | – |
| | Ammonium sulfate | | wt% | – | – | – | – |
| | Osmotic pressure regulator | Sodium chloride | wt% | 5.0 | 1.4 | 4.9 | 2.0 |
| | Trehalose | | wt% | – | – | – | 5.0 |
| | Glucose | | wt% | 0.5 | – | – | – |
| | Adenine | | wt% | 0.1 | – | – | – |
| | myo-inositol | | wt% | 0.2 | – | – | – |
| | Apoptosis Inhibitor | Q-VD-Oph | μM | – | – | – | 0.5 |
| | Propylene glycol | | wt% | – | – | 20 | – |
| Amount of aqueous solution contained in blood collection container | | | mL | 1 | 1 | 1 | 1 |
| Plasma separation composition | | Type | – | A | D | C | A |
| | | Specific gravity at 25°C | – | 1.045 | 1.032 | 1.035 | 1.045 |
| Amount of blood collected | | | mL | 8 | 8 | 8 | 8 |
| Evaluation | Osmotic pressure of mixture solution | | mOsm/L | 442 | 315 | 722 | 345 |
| | Leukocyte residual amount | | % | 1 | 1 | 1 | 3 |
| | | | Determination | ○ | ○ | ○ | ○ |
| | Amount of cfDNA recovered | cfDNA concentration (collection day) | ng/plasma 1 mL | 17 | 19 | 18 | 18 |
| | | cfDNA concentration (storage for 7 days) | ng/plasma 1 mL | 25 | 30 | 24 | 20 |
| | | Increase in cfDNA concentration | ng/plasma 1 mL | 8 | 11 | 6 | 2 |
| | | | Determination | ○○ | ○ | ○○ | ○○ |

[Table 4]

| | | | | Example 9 | Example 10 | Example 11 | Example 12 |
|---|---|---|---|---|---|---|---|
| Aqueous solution | Anticoagulant | EDTA2K·2H₂O | mM | 20 | 20 | 20 | 20 |
| | Water-soluble polymer compound (X) | Dextran (Mn: 40,000) | wt% | – | – | – | – |
| | | Dextran (Mn: 70,000) | wt% | – | – | 5.0 | 5.0 |
| | | Polyethylene glycol (Mn: 3,000) | wt% | – | – | 7.0 | 7.0 |
| | | Polyethylene glycol (Mn: 20,000) | wt% | – | – | – | – |
| | | Polyvinylpyrrolidone (Mn: 10,000) | wt% | – | – | – | – |
| | | Polyvinylpyrrolidone (Mn: 58,000) | wt% | – | – | – | – |
| | Water-soluble polymer compound not corresponding to water-soluble polymer compound (X) | Dextran (Mn: 200,000) | wt% | – | – | – | – |
| | | Polyethylene glycol (Mn: 500,000) | wt% | – | – | – | – |
| | Ammonium sulfate | | wt% | 2.0 | 5.0 | 5.0 | 5.0 |
| | Osmotic pressure regulator | Sodium chloride | wt% | 1 | – | – | – |
| | Trehalose | | wt% | – | – | 3.0 | 3.0 |
| | Glucose | | wt% | – | – | – | – |
| | Adenine | | wt% | – | – | – | – |
| | myo-inositol | | wt% | – | – | – | – |
| | Apoptosis Inhibitor | Q-VD-Oph | µM | – | – | – | – |
| | Propylene glycol | | wt% | – | – | – | – |
| Amount of aqueous solution contained in blood collection container | | | mL | 1 | 1 | 1 | 1 |
| Plasma separation composition | | Type | – | A | A | A | B |
| | | Specific gravity at 25°C | – | 1.045 | 1.045 | 1.045 | 1.055 |
| Amount of blood collected | | | mL | 8 | 8 | 8 | 8 |
| Evaluation | Osmotic pressure of mixture solution | | mOsm/L | 356 | 386 | 396 | 398 |
| | Leukocyte residual amount | | % | 1 | 0.5 | 0.5 | 0.5 |
| | | | Determination | ○ | ○ | ○ | ○ |
| | Amount of cfDNA recovered | cfDNA concentration (collection day) | ng/plasma 1 mL | 17 | 19 | 16 | 17 |
| | | cfDNA concentration (storage for 7 days) | ng/plasma 1 mL | 30 | 27 | 19 | 26 |
| | | Increase in cfDNA concentration | ng/plasma 1 mL | 13 | 8 | 3 | 9 |
| | | | Determination | ○ | ○○ | ○○ | ○○ |

22

[Table 5]

| | | | | Comp. Ex. 1 | Comp. Ex. 2 | Comp. Ex. 3 |
|---|---|---|---|---|---|---|
| Aqueous solution | Anticoagulant | EDTA2K·2H₂O | mM | | 20 | 20 |
| | Water-soluble polymer compound (X) | Dextran (Mn: 40,000) | wt% | | – | – |
| | | Dextran (Mn: 70,000) | wt% | | – | – |
| | | Polyethylene glycol (Mn: 3,000) | wt% | | – | – |
| | | Polyethylene glycol (Mn: 20,000) | wt% | | – | – |
| | | Polyvinylpyrrolidone (Mn: 10,000) | wt% | | – | – |
| | | Polyvinylpyrrolidone (Mn: 58,000) | wt% | | – | – |
| | Water-soluble polymer compound not corresponding to water-soluble polymer compound (X) | Dextran (Mn: 200,000) | wt% | | 5.0 | – |
| | | Polyethylene glycol (Mn: 500,000) | wt% | | – | 7.0 |
| | Ammonium sulfate | | wt% | | – | – |
| | Osmotic pressure regulator | Sodium chloride | wt% | | 2.2 | 2.3 |
| | Trehalose | | wt% | | – | – |
| | Glucose | | wt% | | – | – |
| | Adenine | | wt% | | – | – |
| | myo-inositol | | wt% | | – | – |
| | Apoptosis Inhibitor | Q-VD-Oph | µM | | – | – |
| | Propylene glycol | | wt% | | – | – |
| Amount of aqueous solution contained in blood collection container | | | mL | | 1 | 1 |
| Plasma separation composition | | Type | – | A | A | C |
| | | Specific gravity at 25°C | – | 1.045 | 1.045 | 1.035 |
| Amount of blood collected | | | mL | 8 | 8 | 8 |
| Evaluation | Osmotic pressure of mixture solution | | mOsm/L | 288 | 338 | 341 |
| | Leukocyte residual amount | | % | 20 | 12 | 11 |
| | | | Determination | × | × | × |
| | Amount of cfDNA recovered | cfDNA concentration (collection day) | ng/plasma 1 mL | 18 | 19 | 18 |
| | | cfDNA concentration (storage for 7 days) | ng/plasma 1 mL | 523 | 147 | 121 |
| | | Increase in cfDNA concentration | ng/plasma 1 mL | 505 | 128 | 103 |
| | | | Determination | × | × | × |

**EXPLANATION OF SYMBOLS**

1: Blood collection container
2: Blood collection container main body
3: Plasma separation composition
4: Aqueous solution
5: Plug

**Claims**

1. A blood collection container comprising:

a blood collection container main body;
a plasma separation material contained in the blood collection container main body; and
an aqueous solution contained in the blood collection container main body,
the plasma separation material having a specific gravity at 25°C of 1.027 or more and 1.060 or less, and
the aqueous solution containing an anticoagulant, as well as containing a water-soluble polymer compound
having a number average molecular weight of 500 or more and 180,000 or less, or ammonium sulfate.

2. The blood collection container according to claim 1, wherein the aqueous solution contains the water-soluble polymer compound having a number average molecular weight of 500 or more and 180,000 or less.

3. The blood collection container according to claim 1 or 2, wherein the aqueous solution contains the ammonium sulfate.

4. The blood collection container according to any one of claims 1 to 3, wherein the aqueous solution contains the water-soluble polymer compound having a number average molecular weight of 500 or more and 180,000 or less, and the ammonium sulfate.

5. The blood collection container according to any one of claims 1 to 4, wherein the water-soluble polymer compound having a number average molecular weight of 500 or more and 180,000 or less is dextran, polyethylene glycol, or polyvinylpyrrolidone.

6. The blood collection container according to any one of claims 1 to 5, wherein the water-soluble polymer compound having a number average molecular weight of 500 or more and 180,000 or less is a water-soluble polymer compound having a number average molecular weight of 2,000 or more and 150,000 or less.

7. The blood collection container according to any one of claims 1 to 6, wherein the aqueous solution further contains trehalose.

8. The blood collection container according to any one of claims 1 to 7, wherein the aqueous solution further contains glucose, adenine, or inositol.

9. The blood collection container according to any one of claims 1 to 8, wherein the aqueous solution further contains an apoptosis inhibitor.

10. The blood collection container according to any one of claims 1 to 9, wherein

the blood collection container is a blood collection container in which a predetermined amount of blood is collected, and
when physiological saline in an amount equal to the predetermined amount of blood to be collected in the blood collection container is collected into the blood collection container to obtain a mixture solution in which the physiological saline and the aqueous solution are mixed, an osmotic pressure of the mixture solution is 300 mOsm/L or more.

11. The blood collection container according to any one of claims 1 to 10, wherein the aqueous solution further contains propylene glycol.

12. The blood collection container according to any one of claims 1 to 11, wherein the plasma separation material is a plasma separation composition.

13. The blood collection container according to claim 12, wherein

the plasma separation composition contains an organic component having fluidity at 25°C and an inorganic fine powder,
the organic component contains a resin, and
the inorganic fine powder contains fine powder silica.

14. The blood collection container according to claim 13, wherein the fine powder silica contains hydrophilic silica.

15. The blood collection container according to claim 14, wherein a content of the hydrophilic silica is 0.01 wt% or more and 2.50 wt% or less in 100 wt% of the plasma separation composition.

16. The blood collection container according to any one of claims 13 to 15, wherein the fine powder silica contains hydrophilic silica and hydrophobic silica.

17. The blood collection container according to any one of claims 13 to 16, wherein

the plasma separation composition has a specific gravity at 25°C of 1.050 or more, and
the inorganic fine powder contains an inorganic fine powder having a specific gravity larger than that of the fine powder silica.

18. The blood collection container according to any one of claims 13 to 17, wherein the resin contains a petroleum resin, a cyclopentadiene resin, a polyester resin, or a (meth)acrylic resin.

19. The blood collection container according to any one of claims 1 to 18, wherein the blood collection container is used for separating extracellular free nucleic acid or extracellular vesicle in blood.

20. A method for separating plasma, the method comprising the steps of:

collecting blood in the blood collection container according to any one of claims 1 to 18; and
centrifuging the blood collection container in which the blood has been collected.

21. A method for separating extracellular free nucleic acid, the method comprising the steps of:

collecting blood in the blood collection container according to any one of claims 1 to 19;
centrifuging the blood collection container in which the blood has been collected to separate plasma from the blood; and
separating extracellular free nucleic acid from the separated plasma.

22. A method for separating extracellular vesicle, the method comprising the steps of:

collecting blood in the blood collection container according to any one of claims 1 to 19;
centrifuging the blood collection container in which the blood has been collected to separate plasma from the blood; and
separating extracellular vesicle from the separated plasma.

[FIG. 1]

<table>
<tr><td colspan="2" align="center">**INTERNATIONAL SEARCH REPORT**</td><td>International application No.<br><br>**PCT/JP2022/036449**</td></tr>
</table>

**A. CLASSIFICATION OF SUBJECT MATTER**

*G01N 1/10*(2006.01)i; *G01N 33/48*(2006.01)i
FI: G01N33/48 D; G01N33/48 C; G01N1/10 H; G01N1/10 V

According to International Patent Classification (IPC) or to both national classification and IPC

**B. FIELDS SEARCHED**

Minimum documentation searched (classification system followed by classification symbols)

G01N33/48; G01N1/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| E, X | JP 7169608 B1 (SEKISUI MEDICAL CO., LTD.) 02 November 2022 (2022-11-02)<br>claims, examples, table 2 | 1-22 |
| A | JP 2021-156897 A (SEKISUI MEDICAL CO., LTD.) 07 October 2021 (2021-10-07)<br>claims | 1-22 |
| A | WO 2021/177344 A1 (SEKISUI MEDICAL CO., LTD.) 10 September 2021 (2021-09-10)<br>claims 1, 5, paragraphs [0016], [0020]-[0021] | 1-22 |
| A | JP 2007-271388 A (TSUCHIDA, Hidetoshi) 18 October 2007 (2007-10-18)<br>claims 1, 5-6 | 1-22 |
| A | JP 2017-102114 A (GENERAL ELECTRIC CO.) 08 June 2017 (2017-06-08)<br>claims 1-2 | 1-22 |

☐ Further documents are listed in the continuation of Box C.　　☑ See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **06 December 2022** | **20 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

<div align="center">

**INTERNATIONAL SEARCH REPORT**
**Information on patent family members**

</div>

| International application No. |
| --- |
| **PCT/JP2022/036449** |

| Patent document cited in search report | | | Publication date (day/month/year) | Patent family member(s) | | | Publication date (day/month/year) |
| --- | --- | --- | --- | --- | --- | --- | --- |
| JP | 7169608 | B1 | 02 November 2022 | (Family: none) | | | |
| JP | 2021-156897 | A | 07 October 2021 | WO | 2021/112119 | A1 | |
| | | | | CN | 114340493 | A | |
| | | | | KR | 10-2022-0110437 | A | |
| WO | 2021/177344 | A1 | 10 September 2021 | (Family: none) | | | |
| JP | 2007-271388 | A | 18 October 2007 | (Family: none) | | | |
| JP | 2017-102114 | A | 08 June 2017 | US | 2017/0153223 | A1 | |
| | | | | claims 1-2 | | | |
| | | | | US | 2021/0088503 | A1 | |
| | | | | EP | 3176579 | A1 | |
| | | | | CN | 106893694 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010053180 A1

- WO 2010132783 A1